# EUROPEAN PATENT SPECIFICATION

(11) **EP 1 981 431 B1**
(45) Date of publication and mention of the grant of the patent: **02.07.2014**
(21) Application number: 07717576.8
(22) Date of filing: 06.02.2007
(51) Int. Cl.: A61F 2/00, A61F 13/00

(54) **DEVICE FOR TREATING SLEEP APNEA AND SNORING**
VORRICHTUNG ZUR BEHANDLUNG VON SCHLAFAPNOE UND SCHNARCHEN
DISPOSITIF POUR LE TRAITEMENT DE L'APNÉE DU SOMMEIL ET DES RONFLEMENTS

(30) Priority: 06.02.2006 US 765638 P
(43) Date of publication of application: 22.10.2008
(60) Divisional application: 14168881.2
(73) Proprietor: Linguaflex, Inc., Davie, FL 33330 (US)
(72) Inventor: SANDERS, Ira, North Bergen, NJ 07047 (US)
(74) Representative: Lawrence, John
(86) International application number: PCT/US2007/061721
(87) International publication number: WO 2007/092865

(56) References cited:
- WO-A1-2005/082452
- WO-A2-2005/110280
- US-A1- 2005 092 332
- US-B1- 6 408 851

## Description

### Field of the Invention

This invention relates to devices for maintaining upper airway patency.

### Background of the Invention

Snoring, upper airway resistance syndrome, and obstructive sleep apnea syndrome (OSAS) are all related to narrowing or obstruction of the upper airway during sleep (sleep disordered breathing). According to the National Institutes of Health (NIH), approximately 18 million Americans have sleep apnea (sleep disordered breathing), but fewer than 50% are presently being diagnosed. According to the National Highway Traffic and Safety Administration (NHTSA), 100,000 accidents and 1,500 traffic fatalities per year are related to drowsy driving. More than 50% of Americans over age 65 have sleep difficulties, and prevalence of sleep problems will therefore increase as the over-65 population increases. Each year, sleep disorders, sleep deprivation, and excessive daytime sleepiness add approximately $16 billion annually to the cost of health care in the U.S., and result in $50 billion annually in lost productivity.

### Pathophysiology of sleep disorders

Sleep disorders are largely caused by too much soft tissue in the throat. Humans are unique because their upper airway has a curved shape, an anatomical change that is related to the evolution of human speech. As a result the upper airway of humans is more flexible than that of other species and is more prone to collapse under negative pressure. In the awake state a certain amount of tone is present in upper airway muscles to prevent this collapse. However, during sleep muscle tone decreases in upper airway muscles and in certain susceptible individuals this relaxation allows the airway to collapse (Horner RL. Motor control of the pharyngeal musculature and implications for the pathogenesis of obstructive sleep apnea. Sleep 1996; 19: 827-853).

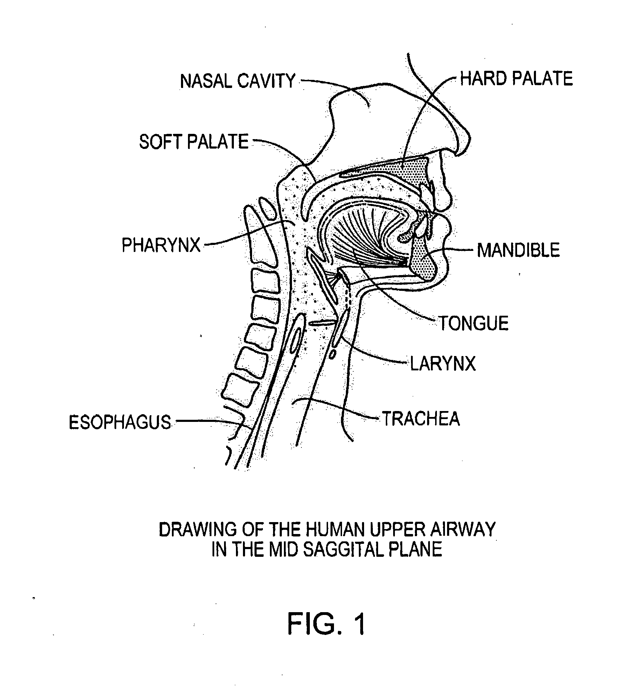

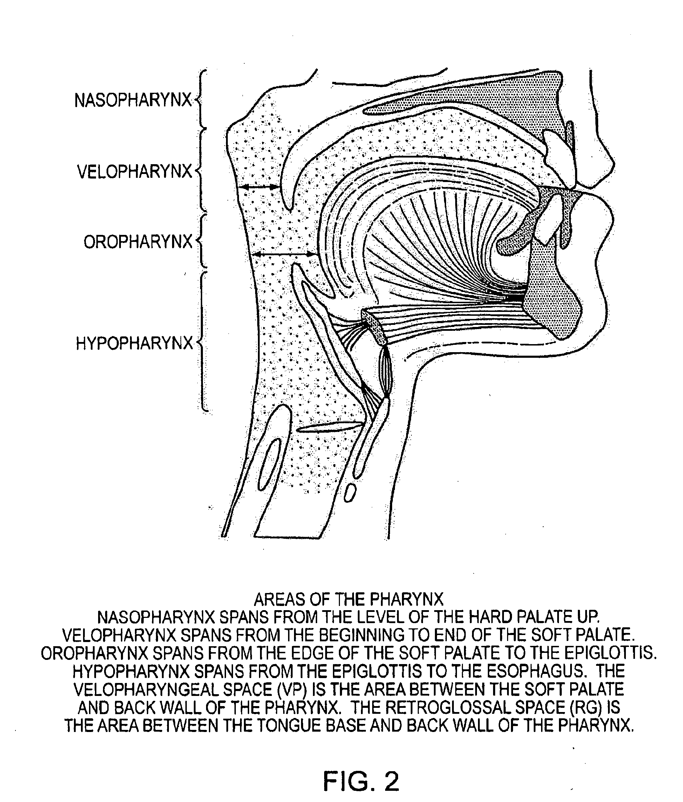

The upper airway refers to the air filled spaces between the nose and the larynx (Figure 1). The most relevant part of the upper airway for sleep disorders is the air cavity at the back of the throat called the pharynx. The pharynx can be divided into three anatomical levels (Figure 2):
1) The *nasopharynx* is the part of the pharynx in the back of the nasal cavity.
2) The part at the back of the mouth is called the oropharynx. To be more precise it is best called the *velopharynx.* This level corresponds to that part of the pharynx containing the velum (soft palate) and tongue curve.
3) The *hypopharynx* is behind the tongue base.

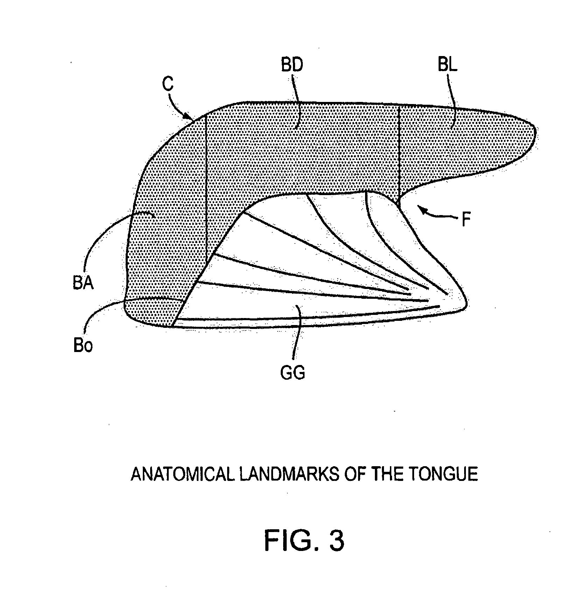

The velopharynx is more susceptible to collapse because there are more soft tissue structures, leaving less room for airflow. The major structures of the velopharynx are the soft palate and the tongue, both of which are very flexible. The soft palate acts as a barrier between the mouth and the nose. In many people it is longer than necessary and extends down between the tongue and pharyngeal wall. The tongue is the largest muscular organ of the upper airway and is anatomically divisible into a blade, body and base (Figure 3). Most of the tongue's curve is at the junction of the tongue body and base.

In the awake condition the structures of the velopharynx maintain their shape because of continuous tone of their internal muscles. When this tone decreases, such as during sleep, these structures become quite flexible and distensible. Without the normal muscle tone that keeps them is place, they tend to collapse at relatively low negative pressures. Although muscles relax throughout the body during sleep many of the respiratory muscle remain active. Specifically the major muscle that pulls the tongue forward, the genioglossus muscle, has been reported to show normal or increased activity during obstructive apneas. Normally the genioglossus is capable of moving the tongue forward and even projecting it out of the mouth. Why the genioglossus muscle sometimes fails to prevent obstructions has not been explained.

During inspiration the chest wall expands and causes negative pressure to draw air into the nose and mouth and past the pharynx into the lungs. This negative pressure causes upper airway soft tissue to deform, further narrowing the airway. If the airway narrows enough the air flow becomes turbulent causing the soft palate to vibrate. The vibration of the soft palate produces the sound known as snoring. Snoring is extremely common effecting up to 50% of men and 25% of women. By itself snoring is not a medical problem although it can be a tremendous problem for the patient's bed partner and a major cause of marital strain.

A small amount of decreased airflow or brief obstructions occur in all humans during sleep. These episodes are counted as medically significant if airflow is decreased more than 50% of normal (hypopnea) or if airflow is obstructed for more then 10 seconds (apnea). The number of apneas and hypopneas that occur during each hour of sleep is measured to diagnose the severity of the sleep disorder. These episodes of hypopnea or apnea often cause some degree of arousal during sleep. Although the patient does not awaken to full consciousness, the sleep pattern is disturbed causing the patient to feel sleepy during the day. If the frequency of hypopnea or apnea is more than 5 episodes an hour it is called upper airway resistance syndrome. These patients often show symptoms related to the sleep disruption. Specifically, these patients are excessively sleepy during the day. In addition more subtle symptoms such as depression and difficulty in concentrating are also commonly reported.

Technically the diagnosis of OSAS. is defined as an average of more than 10 episodes of hypopnea or apnea during each hour of sleep. Although the airway is obstructed the patient makes repeated and progressively more forceful attempts at inspiration. These episodes are silent and characterized by movements of the abdomen and chest wall as the patient strains to bring air into the lungs. Episodes of apnea can last a minute or more, and during this time the oxygen levels in the blood decrease. Finally, either the obstruction is overcome, usually producing a loud snore, or the patient awakes with the feeling of choking.

Very common symptoms in OSAS patients are morning headaches and acid reflux. During airway obstructions the forceful attempts to inspire air can cause tremendous negative pressure in the chest. These high negative can draw acid up the esophagus from the stomach. The acid can travel all the way into the mouth and cause inflammation of the vocal cords and nasal mucosa. The presence of the acid in the upper airway causes reflex bronchoconstriction in the lung that is similar to an asthma attack. If even a small amount of acid enters the lung it can cause the vocal folds to close tightly and itself cause a prolonged apnea called laryngospasm. In many patients the repeated stretching of the espophageal sphincter causes chronic changes and these patients can have acid reflux during the day.

Most importantly, sleep disorders can cause serious medical disorders and death. Apneas cause a large strain on the heart and lungs. Over time the many repeated episodes of apnea cause chronic changes leading to hypertension. Long periods of apnea allow the oxygen levels in the blood to decrease. In turn the low oxygen can cause heart attacks or strokes.

### Treatment Of Sleep Disorders

Although OSAS occurs in both children and adults the cause and treatment is very different. OSAS in children almost always occurs when the child has large tonsils, and tonsillectomy cures the condition. Tonsils naturally decrease in size with age and are rarely a problem in adults. Instead susceptible adults usually have enlargement of their tongues, soft palate and/or pharyngeal walls. This enlargement is mostly due to fat deposits within these structures.

Adult sleep disorders are difficult to treat for a variety of reasons. The upper airway is a very mobile structure that performs the critical functions of swallowing and speech. These functions are easily compromised by surgical procedures or other interventions. In addition, the upper airway also has a large amount of sensory innervation that causes reflex gagging and coughing. Theoretically a physical stent that is placed in the oral cavity and pharynx would be completely effective in relieving sleep apnea. When a patient is totally unconscious, such as when they are anesthetized for surgery, the airway can be stented open by placing a curved oral tube into the mouth and pharynx. In addition, endotracheal tubes establish a secure airway for artificial ventilation. However, after anesthesia wears off, patients immediately sense and react to the foreign objects in their throats and expel them. Therefore devices such as oral and endotracheal tubes, or anything similar, cannot be used for the treatment of OSAS.

Although physical stents cannot be used for OSAS an indirect way of stenting the upper airway with positive air pressure is the most common prescribed treatment for OSAS. This method is called continuous positive airway pressure (CPAP). CPAP requires the use of a mask tightly attached around the nose and connected to a respirator. The exact amount of positive pressure is different for each patient and must be set by overnight testing using multiple pressures. The positive pressure acts like a stent to keep the airway open. CPAP is not a cure but a therapy that must be used every night. Although many OSAS patients are helped by CPAP it is not comfortable for the patient or their bed partner. Patients often cannot tolerate the claustrophobic feeling of a mask tightly attached to their face. In addition they are often many technical problems with maintaining a proper seal of the mask to the face. For these reasons up to half of all patients who are prescribed CPAP stop using it within 6 months (Sanders, "Medical Therapy for Sleep Apnea," Principles and Practice of Sleep Medicine, 2nd Edition, pp- 678-684)

### Tracheotomy

The only completely effective surgical therapy for OSAS is to bypass the entire upper airway by performing a permanent tracheotomy, a surgical procedure that forms a direct connection to the trachea through the neck. This is a dangerous procedure reserved for the worst cases when there is a high risk of serious medical complications from OSAS. Notably, temporary tracheotomies are often performed on patients with severe OSAS to control the airway before performing before any other procedure is performed on their upper airway. The reason is that these patients are at high risk of acute airway obstruction and death if there is any swelling in their airways. Due to the tremendous excess of swollen tissue in their upper airways OSAS patients are very difficult to intubate under emergency conditions. Similarly there is tremendous amount of fat in the neck that makes emergency tracheotomies extremely hazardous.

Prior to current conservative measures, post operative deaths were not uncommon in severe OSAS patients. Moreover these patients often have acclimated to breathing against resistance, and when the resistance is suddenly removed their respiratory drive decreases. Even today the standard of care in treating most OSAS patients is to have them under close observation in an intensive care unit or recovery room after surgical procedures.

### Soft Palate Procedures For Snoring

As the soft palate vibrates more than other tissues it plays a disproportional role in snoring. Various surgical therapies are available that shrink or stiffen the soft palate. The main procedure used is called uvulopalatopharyngoplasty (UPPP). UPPP excises excess soft tissue of the pharyngeal walls and soft palate with a surgical scalpel. Because so much mucosa of the pharyngeal area is traumatized during a UPPP there is a large amount of post operative swelling and severe pain. In selected patients who snore but have no obstructions more limited versions of the UPPP can be done with lasers or electrical cautery.

Newer procedures minimize trauma to the mucosa and use needles to reach the underlying soft tissue to shrink its volume or stiffen it so that it resists vibration. Electrodes can be inserted into the soft palate to deliver radiofrequency energy that shrinks or stiffens the palate (Powell, NB, et al (1998) Radiofrequency volumetric tissue reduction of the palate in subjects with sleep-disordered breathing. Chest 113,1163-1174.) (Somnoplasty; Somnus; Mountainview, CA). Mild caustic agents can be injected that decrease the volume of the soft palate. US patent No. 6439238 to Benzel teaches the application of a stiffening agent to the surface of the soft palate. Most recently, office based implantation of plastic inserts to stiffen the soft palate has been approved by the FDA (Pillar® Procedure, US6546936: Method and apparatus to treat conditions of the naso-pharyngeal area).

The fundamental shortcoming of all procedures that target the soft palate, including the newer techniques, is that they only partially improve OSAS (Loube DI (1999) Technologic Advances in the Treatment of Obstructive Sleep Apnea Syndrome. Chest. 1999;116:1426-1433, Doghramji, K, et al (1995) Predictors of outcome for uvulopalatopharyngoplasty. Laryngoscope 105,311-314). Although studies report a decrease in the number of apneas these patients are rarely cured. Evidently the critical structure causing OSAS is not the soft palate but the tongue.

### Tongue Base Procedures For OSAS

The methods used to treat the tongue base in OSAS are either to permanently decrease its volume, to decrease its flexibility or to move the entire tongue forward.

Surgical excision of the tongue base has been poorly effective. The results for scalpel or laser resection of the tongue base in OSAS treatment have not been good enough to recommend continued application of these procedures (Mickelson, SA, Rosenthal, L (1997). Midline glossectomy and epiglottidectomy for obstructive sleep apnea syndrome. Laryngoscope 107,614-619).

More recently radiofrequency (5,843,021 to Edwards) and ultrasonic (US6,409,720) energy have been proposed to shrink and stiffen the tongue base with radiofrequency energy. The radiofrequency energy is delivered via needle electrodes that are inserted into the tongue base to cause a lesion that scars and shrinks over time. To avoid postoperative swelling and pain a limited amount of lesioning is done in a single session and patients require an average of 5 treatments. About a third of patients have greater than 50% improvement in their OSAS. However, approximately a fourth of patients have significant post operative complications, including, tongue base ulceration and abscesses, and temporary tracheotomy.

A recent introduced device for tongue base advancement is the Repose® system (Influent Corp; San Francisco, CA). The Repose® procedure is performed under general anesthesia, and a screw is inserted at the base of the mandible. The screw contains attachments for a permanent suture that is tunneled under the mucosa of the floor of the mouth to the back of the tongue, then passed across the width of the tongue base, and brought back to attach to a metal hook screwed into the bone of the mandible. The suture is tightened to displace the tongue base forward, and caution must be observed to prevent excess tension leading to necrosis of tissue. Unfortunately studies of the Repose® procedure show that it is ineffective at eliminating OSAS. Only 1 of 15 patients was cured of OSAS while 2 patients had to have the suture removed due to pain and swelling.

More aggressive surgical procedures require reconstruction of the mandible, facial, skeleton or the hyoid bone. An example of the art is US pat. No. 6,161,541 to Woodson that teaches a method of surgically expanding the pharyngeal airway. These procedures require extensive surgery with higher risks and much longer recovery periods.

Other proposed methods for treating the tongue base include stiffening the soft tissue by injection of sclerosing particles US pat. No. 6,742,524 or other implanted material US patent application publication No. 20050004417A1.

### Neuroprosthetic Devices

Various neuroprosthetic devices have been invented that stimulate upper airway muscles. US Pat. No. 4,907,602 to Sanders describes transmucosal stimulation to dilate the airway; U.S. Pat. No. 5,792,067 to Karell teaches an intraoral device that applies electrical stimulation to the hard palate, soft palate or pharyngeal area to induce contraction of the upper airway muscles; U.S. Pat. No. 5,190,053 to Meer teaches an intraoral device that applies electrical stimulation to the genioglossus muscle via electrodes located on the mucosa on the floor of the mouth on either side of the frenulum. In addition U.S. Pat No. 5,591,216 to Testerman describes a totally implantable device to stimulate the nerves to the genioglossus muscles. In addition, WIPO application No 04064729 to Gordon describes a neuroprosthetic device that can be injected into the soft palate to treat snoring. At present these devices have not been clinically proven.

In summary, sleep disorders are a significant health problem without an acceptable solution and there is a need in the art for new and more effective therapies.

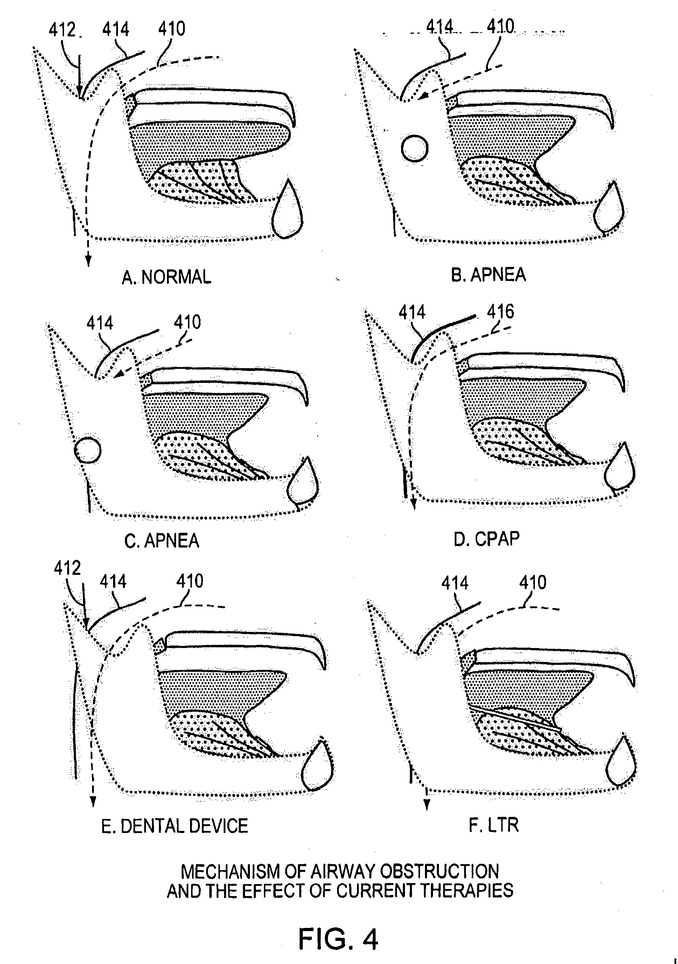

While not wishing to be bound by theory my studies of human tongue anatomy suggest that episodes of obstruction evolve by a sequence of events (Figure 4). The initial inciting event is the deformation of a relatively small part of the tongue. Under certain conditions deformation begins in soft tissue on the top of the tongue, particularly in the area of the tongue curve, and specifically near the center line of the tongue curve. As this tissue deforms it narrows the airway and causes more negative pressure thereby causing greater deformation. This feedback cycle in turn deforms enough tissue in the area to cause a complete obstruction in the velopharyngeal area.

If an initial obstruction occurs near the end of inspiration, the obstruction is relieved by an expiration, or by action of the genioglossus muscle. However, if the obstruction occurs at the beginning of inspiration reflexes trigger stronger inspiratory effort that further lowers airway pressure. This increased negative pressure causes deformation and collapse of most of the tongue base. At this point the airway is firmly plugged by soft issue and activity of the genioglossus only stretches the tongue tissue that is plugged and cannot dislodge it.

Therefore the tongue curve is the critical area that initiates the cascade leading to obstruction. This relaxed muscle is very flexible and easy to deform, however, the converse is also true, very little force is needed to prevent this deformation. Therefore if sufficient counterforce is exerted at the proper localized area of the tongue it can prevent obstruction without noticeable effects on speech and swallowing movements.

How a device could prevent the deformation and collapse of the tongue curve is not a trivial problem:
- This area of the tongue is very mobile during speech and swallowing, therefore the amount of force exerted must be low and highly localized. It is unacceptable to render the area immobile, as would be done if were stiffened by a large implant or scar tissue.
- The whole area of the velopharynx has extensive sensory innervation, and relatively minor stimulation there causes either a gag or a swallow.
- The tongue base and body have a larger blood supply than comparable muscles elsewhere in the body. Any implant placed in the area has a high probability of causing internal bleeding with potentially catastrophic tongue swelling.
- Soft tissue and tongue in particular remodel easily. Specifically sutures or implants that exert force cause the tissue to remodel to relieve that force. This is known as the cheese cutter effect. Therefore the forces applied must be relatively low and applied for limited periods.
- Humans upper airway anatomy is highly variable, and the pathological anatomy of sleep apnea patients is even more variable. Moreover the upper airway anatomy of sleep apnea patients changes over time as the disease progresses or improves.
- Finally, OSAS patients have borderline airways that can obstruct after even minor amounts of swelling such as that following surgical manipulation. Therefore it not obvious how a device could both exert force in the area yet avoid swelling.

Moreover to be maximally effective and get patient and physician acceptance the device would ideally require additional qualities:
- It should be capable of being inserted as an outpatient procedure.
- Preferably the device could be removed during the day and reinserted by the patient at night.
- It would be adjustable to conform to the specific needs of the patient.
- It would be comfortable for the patient.
- When the device was in place it would not be noticeable to anyone else.

International patent publication WO 2005/110280 describes devices and methods for attaching an endolumenal gastrointestinal implant within a patient's digestive tract for treatment of obesity.

WO 2005/082452 discloses a tissue retractor comprising the features defined in the preamble of claim 1.

### Summary of the Invention

There is a tremendous variability in human upper airway anatomy, and even further variation in the pathological changes contributing to sleep apnea and related disorders. Moreover, the pathological anatomy changes over time in each patient as their condition improves or deteriorates. No single method and device is able to treat all contingencies. Therefore there is a critical need for methods and devices that are optimized for different sites in the upper airway.

Embodiments of the invention include devices to prevent or treat upper airway disorders in mammals related to impaired airflow. These disorders are, without limitation, snoring, upper airway resistance syndrome, and obstructive sleep apnea. In addition, this invention is applicable to airway disorders in animals including but not limited to dorsal displacement of the soft palate in horses and brachycephlic obstructive airway syndrome in certain breeds of dog. Those skilled in the art will readily appreciate that application of this invention can be applied to other conditions of the upper airway.

According to the invention, there is provided a tissue retractor for treatment of a breathing disorder as set out in claim 1. Other advantageous features are set out in the dependant claims.

The present invention prevents airway obstruction by preventing the soft tissue of the tongue from deforming. It enlarges the airway when excess tissue is present and also counteracts the deforming influence of negative airway pressure on the relaxed soft tissue of upper airway structures.

My previous PCT publication WO 2005/082452 describes a method and device herein referred to as a Linguaflex tongue retractor (LTR). The LTR consists of a retractor (R), a shaft (S), and an anchor (A). In a preferred example a retractor is physically coupled to the soft tissue of the tongue base. The shaft passes through the midline of the tongue to connect with an anchor. The anchor imparts counterforce through the shaft to the retractor, thereby preventing deformation of the soft tissue.

The present specification describes improvements to the retractor head, shaft and anchor that increase the efficacy of the device while decreasing patient discomfort.

Such an implant is made more comfortable by allowing the device to be under little or no tension during the day, the unloaded state, and to increase the tension to therapeutic levels at night, the loaded state. This increases the comfort for the patient and allows the patient a large degree of control. This is of great importance as the lack of patient compliance is perhaps the largest problem with current sleep apnea therapies.

The shaft of the device can pass deeply into tissue, or pass superficially just beneath the mucosa. The retractor and anchor member is preferably shaped to fit its site so as to distribute force evenly: flat for flat or mildy curved surfaces such as the mid tongue base, pharyngeal wall, and soft palate; wedge shaped for the depths of the pharyngoglossal fold and lateral margin of the soft palate; V shaped for the frenulum; and T shaped for the teeth. The materials of the implant, retractor and anchor could be of any of the well known non-reactive biocompatible materials known in the art. Non-limiting examples of rigid materials include stainless steel, titanium, ceramics, and plastics. Elastomeric materials include silicon and rubber. The force needed to displace the tongue anteriorly or the soft palate superiorly varies from .001 gram to 10,000 grams. More preferable 1 gram to 1000 grams, most preferably 10-100 grams. This force could be applied from .01 sec to permanently. More preferable one minute to 1 month. Even more preferably for the duration of sleep. Most preferably during episodes of restricted upper airway flow.

### Brief Description of the Drawings

The present invention will be more readily understood from the detailed description of exemplary embodiments presented below considered in conjunction with the attached drawings, of which:
Figure 1 is a drawing of the human upper airway in the mid saggital plane;
Figure 2 is a simplified schematic drawing of the tongue and surrounding structures;
Figure 3 illustrates Anatomical landmarks of the tongue;
Figures 4A-4F illustrate a Mechanism of airway obstruction and the effect of current therapies;
Figures 5A-5D illustrate an LTR device;
Figures 6A-6E illustrate a Retractor member;
Figures 7A-7C illustrate a Shaft member;
Figures 8A-8H illustrate an Anchor member, bolster;
Figures 9A-9E illustrate an Anchor member, dental;
Figures 10A-10C illustrate an Anchor member, frenulum area;
Figures 11A-11E illustrate a Frenulum area example;
Figures 12A-12F illustrate a Tongue base implant;
Figures 13A-13L illustrate Tongue base examples;
Figures 14A-14E illustrate The Superior Palatoglossal Fold;
Figures 15A-15F illustrate Pharyngoglossal Fold examples;
Figures 16A-16F illustrate Pharyngoglossal Fold examples.

It is to be understood that the attached drawings are for purposes of illustrating the concepts of the invention.

### Detailed Description of the Drawings

Figure 1. Drawing of the human upper airway in the mid saggital plane.

Figure 2. Simplified schematic drawing of the tongue and surrounding structures
NP, Nasopharynx
VP, Velopharynx
HP, Hypopharynx
SP, Soft palate
P, Hard palate
T, Tongue
GG, Genioglossus muscle

Figure 3. Anatomical landmarks of the tongue. The tongue will be defined as the grey area of this schematic. From front to back the tongue is divded hit a blade, body, and base. The genioglossus muscle (GG) inserts into a connective tissue boundary on the undersurface of the tongue (Bo). The entire region of the genioglossus muscle and its mucosa is referred to as the "frenulum area"
BA) Tongue base
BD) Tongue body
BL) Tongue blade
Bo) Boundary between tongue and genioglossus
C) Tongue curve
F) Frenulum
GG) Genioglossus muscle

Figure 4. Mechanism of airway obstruction and the effect of current therapies.
A) Normal tone in tongue while awake, Tongue remains in position allowing airway to remain open. Blue arrow 410 shows airflow, small black arrow 412 shows the relationship of pharyngeal wall (red line 414) to mandible.
B) During sleep muscle tone is lost in the tongue and it becomes flaccid. Negative pressure in the pharynx during inspiration causes backward collapse of the tongue in the velopharyngeal area because the airway is narrowest at that point and the tongue curve (circle) is most deformable.
C) After the airway obstructs at the velopharyngeal area inspiration lowers the pressure in the pharynx further causing the base of tongue to deform and firmly block the airway.
D) CPAP works by pumping air at high pressure through the nose (thick blue line 416), thereby splinting the pharynx open.
E) Dental devices work by moving the entire jaw forward. As the tongue is attached to the soft tissues along the floor of the mouth, and they attach to the jaw, the tongue is indirectly moved to expand the airway. Note that the jaw has moved in relation to the pharyngeal wall (arrow).
F) The LTR prevents posterior deformation of tongue curve by directly restraining the tongue curve from moving backwards.

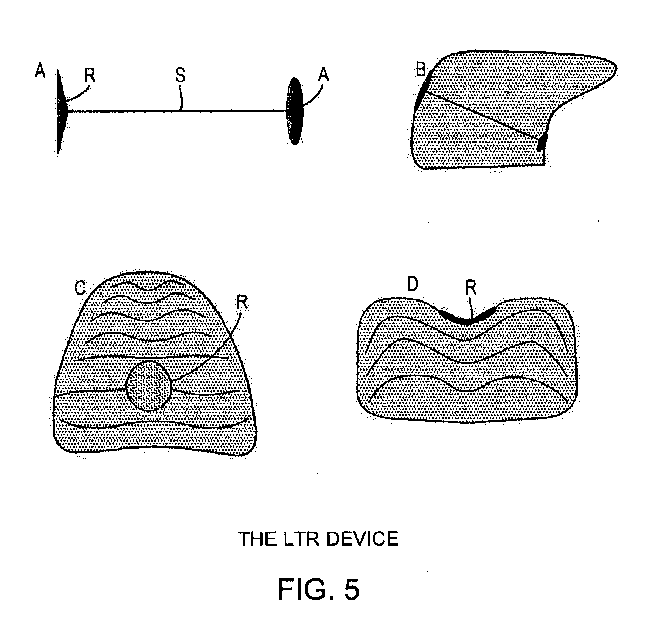

Figure 5. An LTR device. Shown is one example of the LTR.
A. The LTR has three main components: a retractor (R), shall (S), and anchor (A).
B. Side view of the LTR inserted in a tongue.
C. Back view of tongue curve showing retractor position.
D. Back view of tongue base showing the curved midline shape.

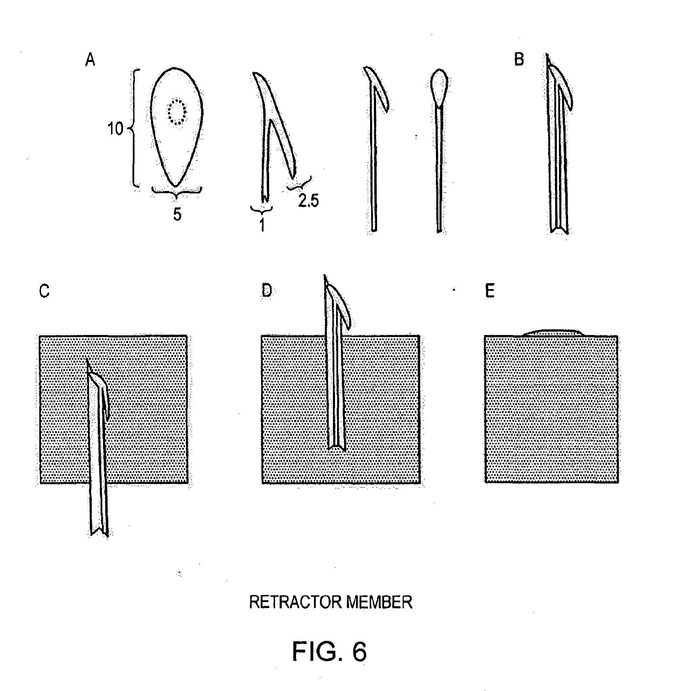

Figure 6. Retractor member. This figure illustrates a retractor component of an LTR that can be mounted on a needle for implantation within upper airway tissue and deploys when the needle is withdrawn. The retractor is shown as an integral component of the shaft and is molded as one piece from soft elastomeric material.
A. Side and front views of the retractor head. The plane of the retractor rests at about 15° relative to the shaft.
B. Side view of retractor head mounted within a needle. A part of the retractor lays on the outer surface of the needle.
C. Side view of needle passing through tissue. Note that the retractor extension lays flat against the needle barrel and does not interfere with passage of the needle through tissue.
D. After the needle penetrates mucosa enough to clear the retractor extension it again extends away from the shaft.
E. Slight traction on the shaft causes the retractor to catch the mucosa and come to rest in its working position.

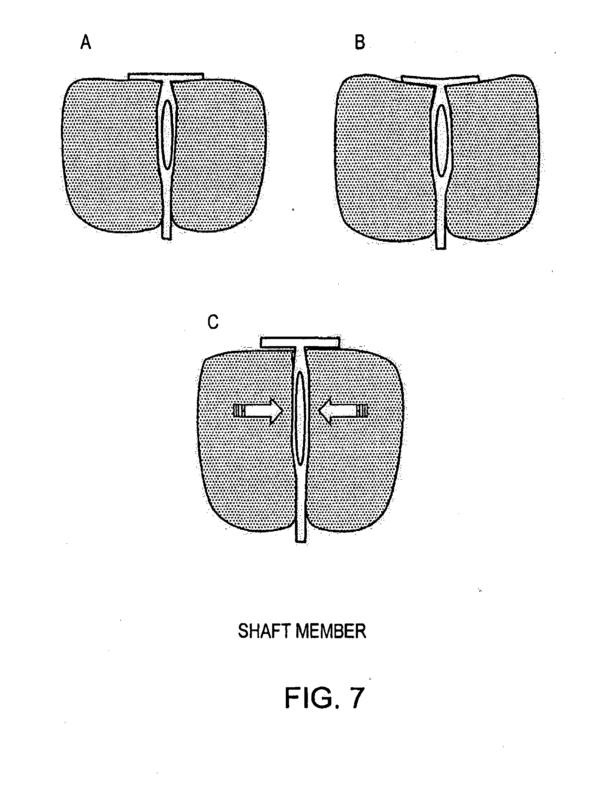

Figure 7. Shaft member. Shown is an improvement to the shaft of an LTR that maintains its retractor tension when the tongue is relaxed, such as during sleep. However, during speech and swallowing, when the tongue base often moves backward, the activity of the tongue squeezes the shaft and thereby lengthens it. In this way there is little or no resistance to the normal tongue movements.
A. Schematic view of LTR in the tongue with normal muscle tone. Note that the retractor lays on the mucosal surface of the tongue base without indenting it.
B. During sleep the tongue loses all tone and tends to flop backward into the airway. The retractor then resists this deformation.
C. During swallowing and speech the tongue base sometimes moves backward. During these movements there is a strong contraction of the tongue muscles. This contraction squeezes the upper shaft, this in turn causes the shaft to lengthen and move the retractor.

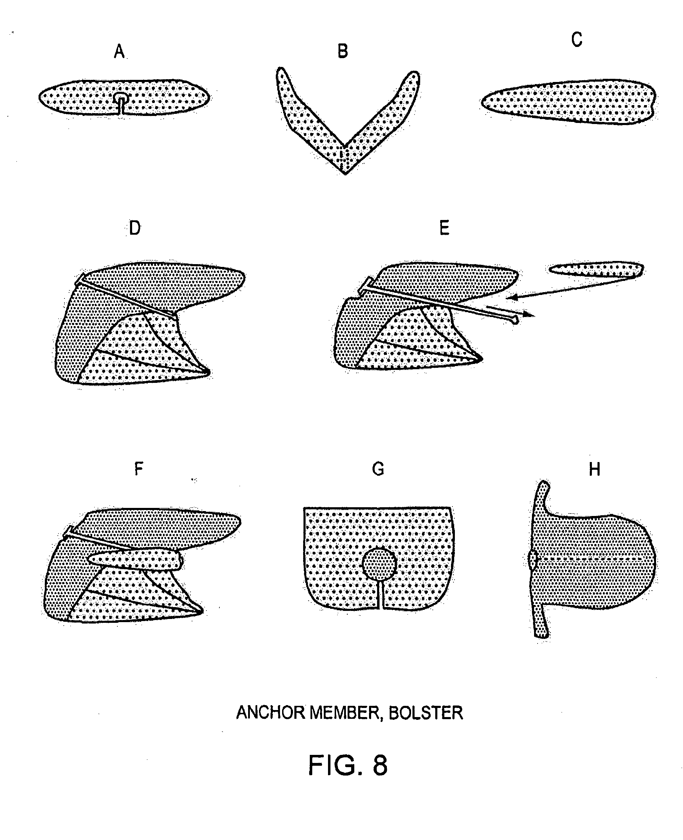

Figure 8. Anchor member, bolster
A. Front view ofbolster.
B. Top view of bolster.
C. Side view of bolster.
D. Side view of tongue with unloaded LTR.
E. Anchor of shaft is pulled forward slotted into cleft on underside of bolster.
F. Bolster in position under tongue.
G. Close up view of LTR anchor sitting in the recess of bolster.
H. Top view of tongue with LTR and bolster.

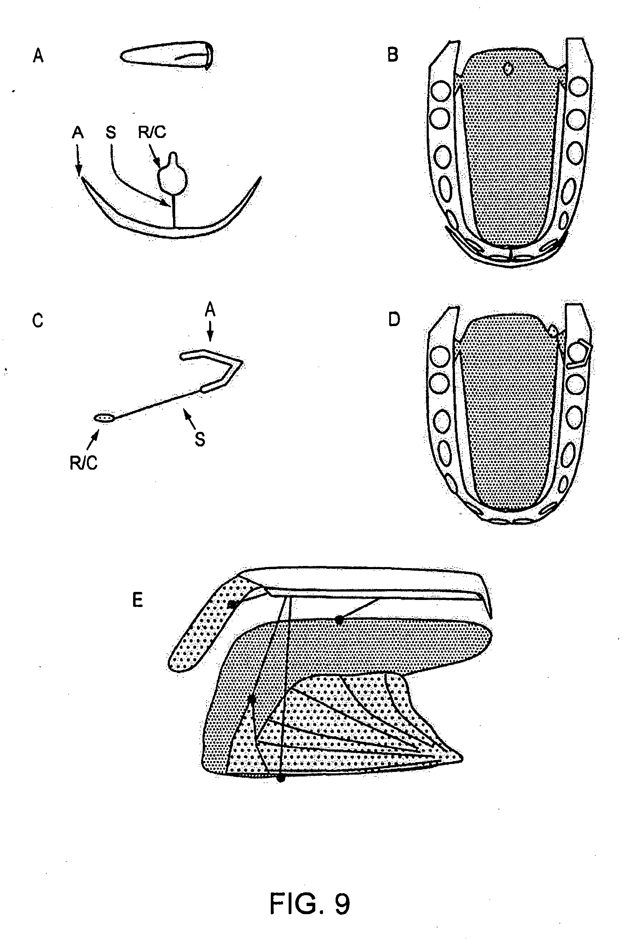

Figure 9. Anchor member, dental
A. A modified anchor that is implanted on the upper or lower front teeth. The anchor (A) interfaces with the teeth, the shaft (S) connects to retractor/coupler (R/C). The retractor/coupler either consists of a retractor that interfaces with tissue, or a coupler component that connects to an implanted retractor, shaft, or anchor member of an implanted LTR.
B. Drawing of top view of tongue and mandible with an LTR implanted from the tongue base to the frenulum. The anchor of the LTR can be reversibly attached to the R/C component of the dental anchor.
C. Another embodiment of a modified anchor for use on the lateral teeth.
D. Top view of tongue and mandible with a lateral dental anchor. The anchor attaches to the molar tooth, the shaft passes through the pharyngoglossal fold, and the retractor rests against the posterior surface of the fold.
E. Palatal prosthesis with some possible coupling extensions for retraction or protraction: the soft palate, PGF, floor of mouth and tongue surface.

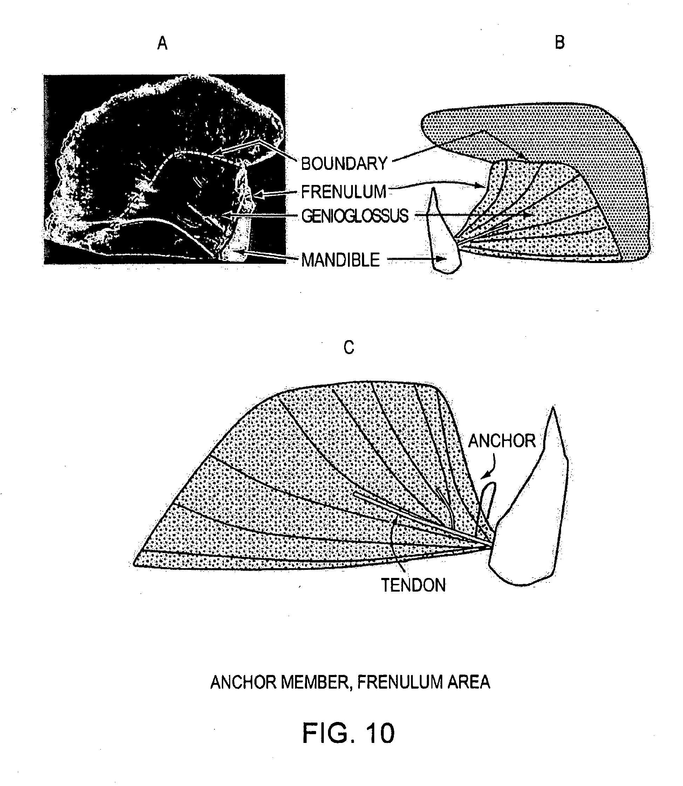

Figure 10. Anchor member, frenulum area.
Within the genioglossus muscle are small tendons upon which muscle fibers insert at various angles. The main tendon is in the middle of the muscle and smaller tendons branch off at various points. Preferably an anchor in the frenulum area is inserted such that its implanted part passes through a tendon. However the anchor can be inserted at any spot in the frenulum area or soft tissue attached to the mandible. The anchor could be coupled to an LTR by a variety of mechanisms as described herein.
A. Side view of the tongue and mandible cut in the centerline (mid-sagittal plane).
B. Drawing of A.
C. Close up of the frenulum area.

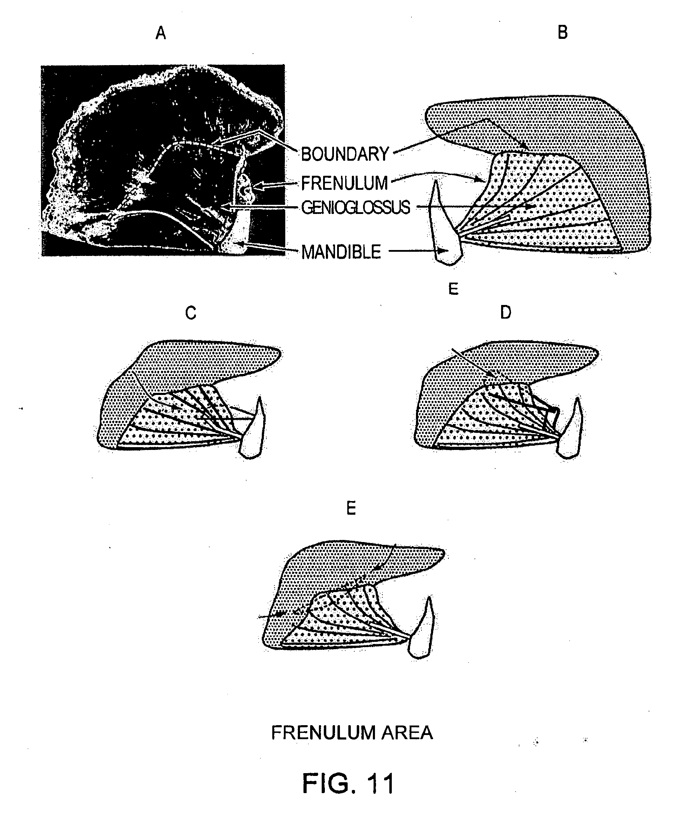

Figure 11. Frenulum area example.
A. Side view of tongue and mandible cut at the centerline. The frenulum is the front edge of the genioglossus muscle, frenulum area refers to the entire genioglossus and surrounding mucosa. The front and rear boundaries of the genioglossus muscle are marked by solid lines. The genioglossus muscle attaches to a small area on the inner surface of the mandible and tendinous extensions from that area. It fans out from these attachments to insert mostly into connective tissue along the length of the body and base of the tongue called the boundary layer.
B. Drawing of A.
C. Shown is an LTR passing through the frenulum area and anchored externally to a dental anchor. The implanted part of the LTR exerts side forces on the genioglossus muscle fascicles and this is conveyed to the boundary layer and finally to the tongue base (arrow).
D. Shown is an LTR passing through the boundary layer and anchored to a frenulum anchor. Displacement of the tongue is marked by the arrow.
E. Shown is a fully implanted LTR in the frenulum area connecting the boundary area in two places. Note that the beneficial retraction of the tongue base causes some retraction of the tongue blade, however, this does not interfere with tongue function.

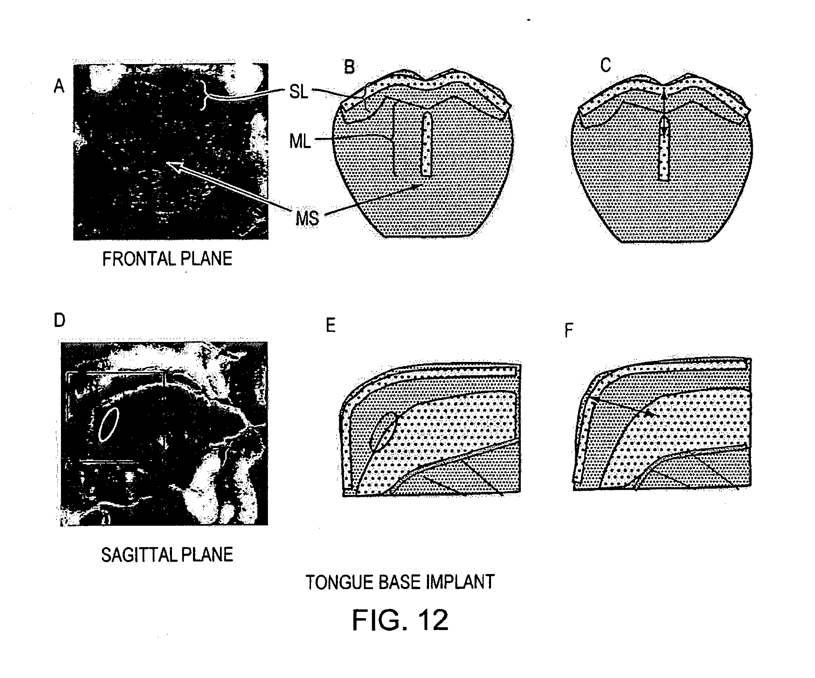

Figure 12. Tongue base implant.
A. Frontal section of tongue base.
B. Drawing of A. Light lines are the connective tissue of the tongue superior layer (SL) and midline septum (MS). ML, middle layer.
C. Position of LTR implant connecting SL and ML.
D. Tongue seen in mid-sagittal plane. Oval marks the area of mechanical decoupling.
E. Schematic drawing of the box marked in D.
F. Position of implant.

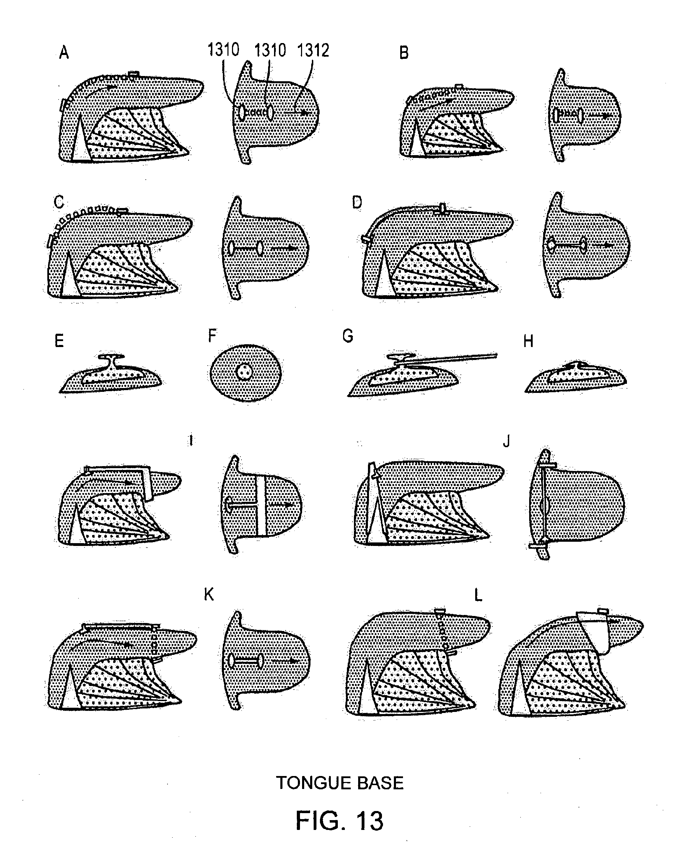

Figure 13. Tongue base examples
A. Lateral (left) and top (right) drawings of the tongue with an LTR connected by a shaft passing underneath the tongue base mucosa. Green ovals 1310 correspond to anchors and retractors, shaft is dotted yellow when implanted and solid when outside, black arrows 1312 show direction of traction.
B. An LTR with the shaft taking a more direct route between retractor and anchor.
C. An LTR with the shaft exiting from mucosa close to the retractor and anchor.
D. Implanted anchor and retractor with a reversible attachable shaft.
E. Lateral view of a partially implantable anchor or retractor.
F. Top view of a partially implantable anchor or retractor.
G. Partially implantable anchor/retractor showing the shaft connection.
H. Lateral view of a partially implantable anchor/retractor with the extension depressed flush with mucosa when not in use.
I. An LTR with an elastic sleeve placed over the tongue blade and a shaft connecting to a semi-implanted retractor member.
J. An LTR anchored at the PGFs and a shaft passing across the tongue base and connecting to a semi-implanted retractor member.
K. An LTR anchored beneath the tongue blade with a shaft passing through the tongue blade to a intermediate anchor on the superior surface of the tongue. The shaft then passes posteriorly to a semi implanted retractor member. This allows adjustment of tension from the anchor site beneath the tongue blade.
L. Left, a rigid shaft connects an anchor member below the tongue blade to a retractor member above the tongue blade. The retractor member is rotated forward by a sleeve that is reversibly placed over the tongue blade. The rotation of the retractor member, along with the rigid shaft, displaces the tissue of the tongue base along the midline. Drawing is intentionally exaggerated to show the effects.

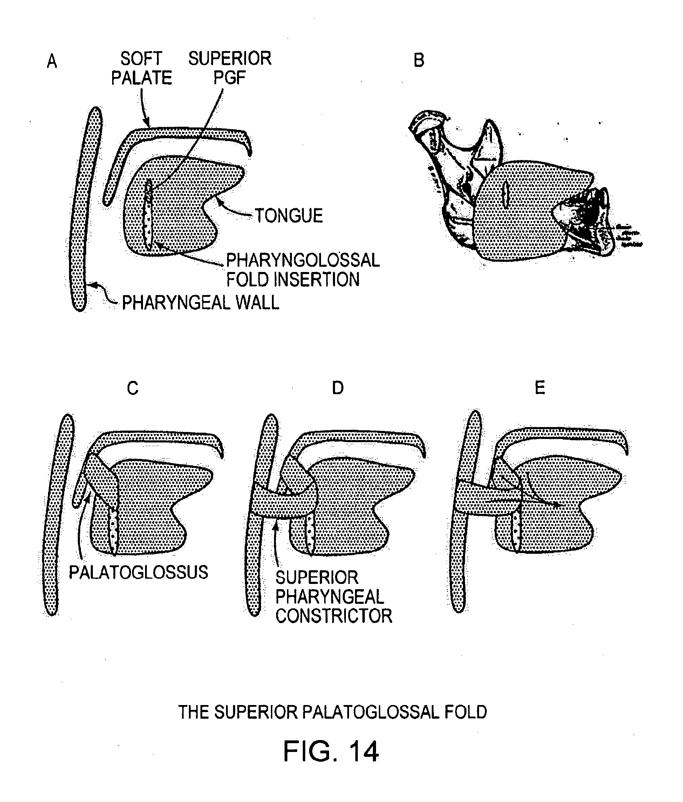

Figure 14. The Superior Palatoglossal Fold.
A. Side view of the upper airway showing the area of the tongue where the PGF inserts. A smaller superior region is of particular significance as it receives overlapping insertions of muscles connecting to the soft palate and lateral pharyngeal walls, including but not limited to the palatoglossus and superior pharyngeal constrictor muscles.
B. Side view of tongue in reation to mandible with the area of superior PGF attachment marked.
C. The palatoglossus muscle is shown connecting the soft palate to the superior PGF.
   The superior pharyngeal constrictor muscle connects the pharyngeal walls to the superior PGF.
D. Schematic showing that retraction force of the PGF is dispersed to the tongue base, soft palate, and lateral pharyngeal walls.
E. Schematic showing that retraction force of the PGF is dispersed to the tongue base, soft palate, and lateral pharyngeal walls

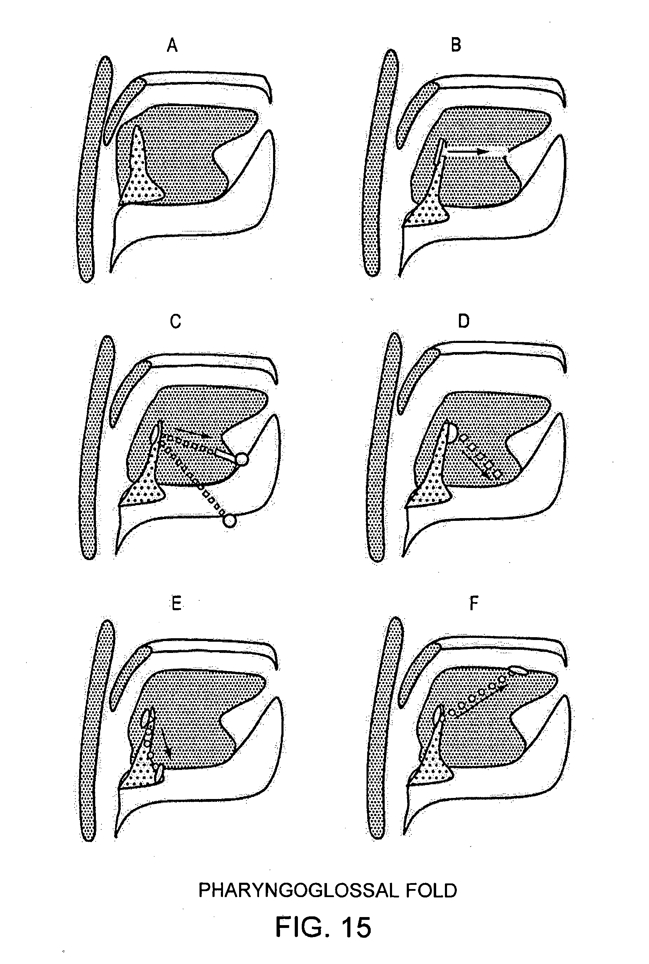

Figure 15. Pharyngoglossal Fold examples
A. Drawing showing the posterior collapse of the tongue and its effects on the airway.
B. A retractor at the PGF and a shaft that passes across the frenulum to a retractor in the other PGF.
C. A retractor in the PGF and a shaft passing through tongue tissue to emerge and connect to a modified anchor. An. alternative embodiment passes through the floor of mouth to an external anchor resting on the skin.
D. An implanted LTR with a retractor in or near the PGF and a shaft passing through tongue to an anchor implanted in genioglossus muscle, or floor of mouth structures.
E. An LTR with a retractor in the superior PGF and an anchor in the inferior. PGF.
F. A retractor in the PGF and a shaft passing through tongue to an anchor on the superior surface of the tongue.

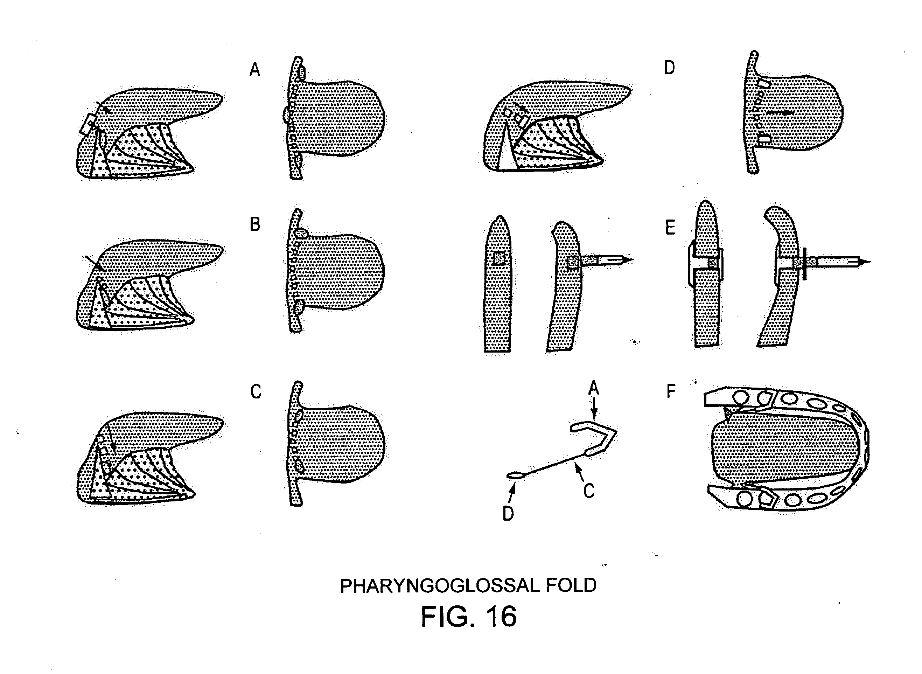

Figure 16. Pharyngoglossal Fold examples.
A. Retractor at tongue base connected by two sub-mucosal shafts to anchors in front of each POP.
B. A sub-mucosal shaft connect two retractor/anchor members in front of each PGF.
C. Two implanted retractor/anchor members in or near the PGFs are connected by a sub-mucosal shaft.
D. Magnets implanted in or near each PGF are connected by a sub-mucosal shaft.
E. Left, a magnet implanted in a PGF is retracted by a magnet of opposite polarity attached to a modified anchor. Right, a magnet is enclosed in an implant that is has two flanges to keep in place within the PGF.
F. Left, a schematic of a dental type modified anchor. The anchor member is a clasp that reversibly attaches to teeth as shown. on right. A shaft of variable length attaches to a retractor member or a coupling mechanism that in turn connects to a an implanted LTR. The retractor member may be a magnet or mechanical mechanism. Right, Drawing of tongue and mandible seen. from above, Two embodiments of the dental modified anchor are shown: bottom, the retractor member is a magnet that couples to an implanted magnet as shown in E left; top, the shaft ends with a magnet that couples to a reversible magnetic implant as shown in E, right.

### Detailed Description

The term "subject" as used herein includes animals of mammalian origin, including humans. Anatomical terminology used to describe position and orientation as used herein can best be defined by the following description:

When referring to animals, that typically have one end with a head and mouth, with the opposite end often having the anus and tail, the head end is referred to as the cranial end, while the tail end is referred to as the caudal end. Within the head itself, rostral refers to the direction toward the end of the nose, and caudal is used to refer to the tail direction. The surface or side of an animal's body that is normally oriented upwards, away from the pull of gravity, is the dorsal side; the opposite side, typically the one closest to the ground when walking on all legs, swimming or flying, is the ventral side. On the limbs or other appendages, a point closer to the main body is "proximal"; a point farther away is "distal". Three basic reference planes are used in zoological anatomy. A "sagittal" plane divides the body into left and right portions. The "mid-sagittal" plane is in the midline, i.e. it would pass through midline structures such as the spine, and all other sagittal planes are parallel to it. A "coronal" plane divides the body into dorsal and ventral portions. A "transverse" plane divides the body into cranial and caudal portions.

When referring to humans, the body and its parts are always described using the assumption that the body is standing upright. Portions of the body which are closer to the head end are "superior" (corresponding to cranial in animals), while those farther away are "inferior" (corresponding to caudal in animals). Objects near the front of the body are referred to as "anterior" (corresponding to ventral in animals); those near the rear of the body are referred to as "posterior" (corresponding to dorsal in animals). A transverse, axial, or horizontal plane is an X-Y plane, parallel to the ground, which separates the superior/head from the inferior/feet. A coronal or frontal plane is an Y-Z plane, perpendicular to the ground, which separates the anterior from the posterior. A sagittal plane is an X-Z plane, perpendicular to the ground and to the coronal plane, which separates left from right. The mid-sagittal plane is the specific sagittal plane that is exactly in the middle of the body.

Structures near the midline are called medial and those near the sides of animals are called lateral. Therefore, medial structures are closer to the mid-sagittal plane, lateral structures are further from the mid-sagittal plane. Structures in the midline of the body are median. For example, the tip of a human subject's nose is in the median line.

Ipsilateral means on the same side, contralateral means on the other side and bilateral means on both sides. Structures that are close to the center of the body are proximal or central, while ones more distant are distal or peripheral. For example, the hands are at the distal end of the arms, while the shoulders are at the proximal ends.

### Definitions

"Anchor" refers to a component of the device that mechanically couples to a site that is immobile relative to the retractor.

"Deformation" refers to an abnormal change in the shape of upper airway soft tissue structures. This deformation can be due to negative pressure acting on relaxed upper airway structures during sleep causing them to narrow the upper airway. Most preferably this soft tissue can be the tongue curve.

"Frenulum" refers to the vertical anterior edge of the genioglossus muscle. The frenulum passes from the floor of the mouth up to the centerline of the underside of the tongue. The frenulum marks the boundary between the tongue blade and tongue body. "Frenulum area" refers to the genioglossus muscle and its surrounding mucosa.

"Loaded" refers to an LTR that can have its tension adjusted such that it has minimal tension during the day and higher therapeutic levels of tension at night. The loaded configuration corresponds to the higher therapeutic levels.

"Modified anchor" is an additional component that allows attachment of the permanent anchor of the LTR. In some embodiments the modified anchor allows the patient to adjust tension in the LTR, Specifically to increase tension at night and release it during the day.

"Palate retractor" refers to a complete device used for the prevention soft palate deformation.

"Permanent anchor" refers to an anchor component of an LTR that remains on the LTR for the duration of the implantation. The permanent anchor prevents the anterior end of the shaft from slipping back into tongue tissue. In certain embodiments the permanent anchor also serves as a part of a connector when a "modified anchor" is used.

"Pharyngeal wall retractor" refers to a complete device for the prevention of pharyngeal wall deformation.

"Protract" means to lengthen or push apart.

"Reverse deformation" refers to a change in soft tissue shape caused by the tissue retractor. In some embodiments reverse deformation refers to restoring a deformed structure to its normal shape. In other embodiments reverse deformation refers to an indentation of soft tissue in a given area due to the action of a tissue retractor.

"Sleep breathing disorders" refers to all breathing disorders occurring during sleep including but not limited to obstructive sleep apnea, obstructive sleep apnea syndrome, upper airway resistance syndrome, and snoring.

"Tongue base" refers to the part of the tongue posterior to the tongue curve. In anatomical terms the line of demarcation of the tongue base is the circumvalatte papillae, a grossly visible line of raised taste organs on the superior surface of the tongue.

"Tongue blade" refers to the part of the tongue anterior to the frenulum. It is covered by mucosa on its top, sides and undersurface.

"Tongue body" is the mid part of the tongue located between the tongue blade and tongue base.

"Tongue boundary" or "boundary" is the inferior surface of the tongue body and base. The genioglossus muscle inserts onto a large part of the boundary.

"Tongue curve" refers to the area of the tongue where its superior surface curves from a horizontal orientation (tongue body and blade) to a vertical orientation (tongue base).

Preferably tongue curve refers to the soft tissue in this area between the mucosal covering of the tongue and the connective tissue boundary where the genioglossus muscle attaches.

"Tissue retractor" refers to the complete device of embodiments of the invention for the prevention of soft tissue deformation. The device may be used without limitation in the tongue, soft palate, or pharyngeal walls.

"Tongue retractor" refers to a complete device used for the prevention of tongue deformation. Preferentially it comprises a retractor connected to a shaft which in turn is connected to an anchor.

"Laryngeal retractor" refers to a complete device for the prevention of laryngeal soft tissue deformation.

"Retractor" or "retractor head" or "retractor member" refers to a part of the overall tissue retractor. The retractor physically interacts with soft tissue, either directly or indirectly, to prevent it from deforming. In certain embodiments the retractor head is a disc located on the external surface of the tongue, in other embodiments the retractor head is an inflatable balloon, in other embodiments the retractor head may have curved parts that act like hooks, in other embodiments the retractor head may be a flexible wire passing through the tissue. In some embodiments it may be totally implanted within tissue.

"Retractor shaft", "shaft" or "retractor member" refers to that part of the tongue retractor that attaches to the retractor head and serves to connect it to the retractor anchor. In some embodiments the retractor In different embodiments the shaft may be rigid or flexible, solid or hollow, one piece or multiple linked pieces.

"Unloaded" refers to an LTR that exerts little or no tension. As used herein this is usually meant as the configuration during the day. In comparison, the LTR is loaded to therapeutic levels at night.

### Examples

### 1. Retractor member (Figure 6)

Disclosed here is a retractor member that is inserted by a needle and automatically deploys to its working shape.

The retractor head prevents the tongue base from deforming. The preferred qualities for a retractor head that rests upon tongue base mucosa are that its depth is minimal so that it is not noticeable to the patient yet its surface area is large enough to provide sufficient counterforce. Integral to its design is the delivery device used to insert the LTR. It is preferable that the entire device be inserted from the anterior tongue with minimal instrumentation used at the back of the tongue. Therefore the retractor head preferably automatically deploys to its working shape after being implanted by a needle inserted from the front of the tongue.

This example design of the retractor head allows it to be easily inserted, for example by a needle. The retractor head may fold within a needle but deploy to its working shape after insertion. Many mechanisms are known that allow a device to be minimized for insertion in the body, non-limiting examples include nitinol wire, high pressure balloons, and spring mechanisms. These mechanisms work well but add complexity and unnecessary expense.

The retractor component may be oval shaped (10 mm long, 5 mm wide, 2.5 mm deep) and is molded together with the shaft (1 mm) as a single piece from moderate consistency medical grade silicon (Shore 80 durometry, Nusil. Ca) (Figure 6A). The retractor is tilted 75° in relation to the shaft. When the device is threaded into a needle (6B) one side of the oval extends out of the needle port and projects at a 15° angle relative to the outside wall of the needle. When the needle is inserted through tissue this extension is pushed flush against the needle wall and causes a minimum increase in the needle's profile (6C). However, immediately after the needle passes through mucosa the retractor reverts to its extended position (6D). In this manner pulling back the needle causes the retractor to catch mucosa and prevents it being withdrawn along with the needle. After the needle is removed, minor tension on the shaft causes the retractor head to rotate into proper position and lay flush against the mucosa (6E).

The practical advantages of this is that the physician can rapidly and easily insert and withdraw the needle and the device automatically settles into its proper position.

### 2. Shaft member (Figure 7)

Disclosed here is a modified shaft that adapts its length to avoid interfering with normal movements of the tongue base.

The counterforce exerted against the back of the tongue base is preferably present during sleep but not during the awake state. More preferably, the counterforce is present when the tongue is relaxed and vulnerable to posterior collapse, but not during speech and swallowing. During swallowing the tongue base moves rapidly backward about 1 cm to contact the back wall of the pharynx. The tongue base moves similarly during some speech movements, albeit with much less force. It is desirable that these swallowing and speech movements are not impaired.

In one embodiment of the shaft the section within the tongue is distensible, one non- limiting example being a balloon. Compression of the balloon portion of the shaft allows the shaft to lengthen. During swallowing the tongue contracts forcefully around the shaft. This contraction squeezes the balloon and lengthens it, thereby displacing the retractor head superiorly. As the tongue base moves superiorly in the area of the retractor head during swallowing, the compression exerted on the shaft causes the shaft to lengthen proportionally and prevents the retractor head from exerting unneeded counterforce on the tongue base during swallowing. However, the ability to exert the proper amount of counterforce when the tongue is relaxed is maintained, The amount of distensibility is preferably .01 to 10 cm, more preferably 1 cm.

This embodiment is a preferred but non-limiting example of the invention. The decrease of counterforce by the retractor head during swallowing and speech can be accomplished by many mechanical and electromechanical mechanisms known. Those skilled in the art can readily appreciate that the invention can have multiple embodiments.

### 3. Anchor member: bolster, dental, implanted (Figures 8,9,10)

Disclosed here are modified anchors that allow reversible loading of an implanted LTR.

The anchor is the anterior component of the LTR that resists displacement of the shaft and retractor head. In a preferred embodiment the LTR is under little or no tension during the day (unloaded state) and is adjusted to exert tension at night (loaded state). In this embodiment the anchor merely prevents the anterior end of the shaft from being pulled back into tongue tissue. For this purpose a small flange is sufficient. However, at night when further retractor counterforce is desired, the anchor can be replaced, modified, or supplemented; collectively referred to as a modified anchor.

One embodiment of a modified anchor is a bolster that is interposed between the permanent anchor and the tongue. This bolster either lengthens the shaft, or if the shaft is set at a fixed length it increases the total volume compressed between retractor head and shaft. In either case the addition of the anchor causes a reversible increase in retractor counter/force.

In one embodiment of the modified anchor the bolster is composed of silicon gel shaped as a V (Figure 8. A, B, C). The concave inner surface of the 'V adapts to the wedge shape of the frenulum, the structure underneath the tongue blade. The intent is to spread the retracting counterforce across a wide surface area. In the center of the anchor bolster is a conduit through which the permanent anchor and shaft is threaded. In one embodiment there is a cleft beginning in the center of the top center edge. This cleft is about the width of the shaft but less then that of the permanent anchor. The patient can reach under the tongue and pull the permanent anchor forward (Figure 8. D, E, F), slip the bolster under the tongue, lay the shaft into the cleft, and release the permanent anchor. The permanent anchor then securely rests against the front surface of the cleft and exerts force. The cleft may be reinforced with a harder grade of silicon or another biocompatible material.

Another option is to secure the permanent anchor to a modified bolster that is permanently or reversibly attached to the teeth, a dental anchor (Figure 9). Many devices that attach to teeth are known in the art. A non-limiting example is 'T' shaped; the top cross bar of the T rests against the front surface of the lower incisor teeth. The initial section of the vertical line of the T is thin enough to pass between the front two incisor teeth. This vertical part widens to allow the retractor head to be threaded. The final part of the T narrows again to about the width of the shaft. This mechanism allows the anchor to be easily and reversibly attached to the dental bolster (Figure 9. A, B).

Another example is a dental anchor optimized for use on the sides of the mouth rather then the front. This example (Figure 9. C, D) anchors to a molar or premolar tooth or neighboring structures. This example is advantageous due to the short distance between the LTR and the modified anchor, its position on the lateral aspect of the tongue is unlikely to interfere with normal tongue function, and it easily accessible for placement, adjustment and removal by patient and physician.

In a further example a dental prosthesis is used as an anchor that couples to LTRs in the soft palate, palatoglossal folds, pharyngoglossal folds, tongue, or other upper airway sites (Figure 9. E). These prostheses are well known in the dental arts, and provide a wide and stable platform for anchoring the LTR. Further examples can take advantage of the large size and position of these prostheses. Those skilled in the art can understand that a variety of electrical or mechanical mechanisms could be incorporated within these prosthesis. As a non-limiting example, electrical motor could be used to control the force applied to coupled LTRs at multiple locations in the upper airway.

Still another example of a modified anchor is partially implanted into the floor of the mouth. In one example a puncture is made across the frenulum or soft tissue structures of the floor of the mouth. A flexible shaft is threaded through the puncture and the ends connected to make a ring like structure. This modified anchor therefore is securely fixed within tissue while the remainder lies along the floor of the mouth (Figure 10). The modified anchor then can be reversibly attached to the permanent anchor at night and disengaged in the morning.

### 4. Frenulum Area

Disclosed are example methods and devices for retracting or preventing deformation of the tongue base by retracting the genioglossus muscle or the boundary fascia upon which genioglossus muscle inserts (collectively referred to as the frenulum area).

It has been unexpectedly found that the shaft of the LTR can be safely passed across the undersurface of the tongue. This tissue contains the genioglossus muscle and its anterior edge is the frenulum (Figure 11. A, B). The genioglossus muscle originates from the mandible and has multiple separate muscle fascicles that fan out from a horizontal to vertical angle. The genioglossus fascicles attach to a layer of connective tissue within the tongue called the boundary (Figure 11 A, dotted line). The genioglossus fascicles normally act by exerting force in the axis of the fascicle onto the part of the boundary to which they are attached. However, even when inactive the fascicles are mechanically coupled to the tongue boundary and can exert force if passively moved. Unexpectedly, this can be done by pulling these fascicles perpendicular to their axis (Figure 11. C). To simplify the mechanism, the genioglossus fascicles are lassoed by the LTR shaft.

There are certain important considerations in placing a retractor through the genioglossus: First, the genioglossus is soft in comparison to the tongue base, therefore too much force applied in a localized area can tear the tissue or cause undesirable tissue remodeling over time, sometimes called the "cheese cutter effect". However, there is a central tendon to the genioglossus that is very strong. This tendon is located approximately 1 cm from the edge of the frenulum. Second, the nerve supply to the genioglossus passes along the superior aspect of the muscle, therefore the top .5 cm of the muscle, the area directly below the tongue blade, is not a preferable site for the implant.

In one example the shaft is a 5 cm length of elastomeric material that is ribbon shaped. The cross sectional dimensions are .5 mm depth and 3 mm width. The wider dimension of the ribbon will exert the force on tissue as its force is disperses over a wider area then the narrow edge of the ribbon. The shaft is attached to a needle and passed through the muscle approximately 1 cm behind the frenulum. The ends of the shaft are then reversibly coupled to a modified anchor. The middle section of the shaft itself exerts anterior retracting force onto the genioglossus muscle and acts as a retracting head: one or both ends of the shaft can then be brought forward and secured to a modified anchor. This displacement is transmitted to the tongue base causing some degree of concavity. The passive movement is preferably in an anterior and inferior direction.

The advantages of genioglossus muscle retraction is that this muscle group is easily accessible beneath the tongue. The tissue is soft and easily compressed, making it easy to pierce without complications. The position under the tongue is invisible to others, a quality important for the patient.

A further option is to pass the LTR deeper into the tongue to couple directly to the boundary layer (Figure 11. D). The boundary layer is a relatively firm connective tissue structure which spans the length of the body of the tongue and receives the insertion of the genioglossus muscle. The advantages of coupling to the boundary layer are that it provides a more secure attachment then the genioglossus itself. However, greater care is needed for placement of the device. Specifically the lingual arteries course just superior and lateral to the boundary layer so it is essential that the insertion be made medial to this structure.

In a further alternative a fully implanted LTR connects one site that effects the tongue base and is anchored at another site that does not. A non-limiting example is shown in Figure 11 E. Here the posterior boundary layer is coupled to the anterior boundary layer. Tension between the two sites displaces the tongue base forward. Simultaneously there is some displacement force exerted around the anterior boundary site but this has insignificant effects on normal tongue function.

### 5- Implanted Tongue Base Retractor (Figure 12)

Disclosed here are example methods and devices that are implanted within the tongue and exert highly localized forces to prevent mechanical decoupling of tongue base structures.

Chronic implants within the tongue are technically challenging and potentially dangerous. The tongue is a mobile structure and tongue movements during swallowing and speech are dependent on this mobility. The tongue has no bones within it and its mechanism of movement is unique among the muscular structures of the body. Most skeletal muscles are attached to bones and movement occurs as mechanical levers. In the tongue structures cause movement by expanding and changing their shape and volume. The mechanism is called a muscular hydrostat and is can be likened to a flexible hydraulic system. In addition the tongue has extensive nerve and blood supply that can be easily damaged. Moreover, the tongue has a tremendous ability to remodel itself when effected by implants and other forces. This is why many prior art devices have failed due to gradual loss of tension or extrusion. Moreover any implant is a potential site for infection and scarring. For these reasons any invasive intervention in the tongue must be designed with a detailed knowledge of tongue anatomy and physiology.

Therefore the implanted embodiments herein are carefully designed to be as minimally invasive as possible and to focus there effects on the most critical areas of pathology without risking interference with normal function.

A preferred example of an implanted LTR disclosed here is a very minimal device implanted into the tongue-base.

The tongue is covered by mucosa and this mucosa has underlying connective tissue. The connective tissue is thickest below the superior surface of the tongue. This superior surface is intimately connected to the underlying superior longitudinal muscle. Together the mucosa connective tissue and muscle form a superior layer (SL) that spans the superior surface of the tongue from the tongue tip to its base. This superior layer is normally coupled to the underlying middle layer of the tongue which is largely composed by the transverse muscle. The transverse muscle originates from a fascial sheet called the medial septum (MS) oriented in the centerline of the tongue (mid-saggital plane).

Although not wishing to be bound by theory studies by the inventor suggest that the vibration during snoring and the stretching during airway obstruction gradually loosen the attachment of the superior layer to the middle layer. This is reflected by the widening of the superior layer in the area of the tongue curve, marked by an oval. This mechanical decoupling results in a more flaccid and compliant tongue base that deforms more easily when the pressure in the airway decreases, thereby making the patient susceptible to sleep apnea and other sleep breathing disorders.

In one example a very small LTR can be inserted at the curve of the tongue base to correct the mechanical decoupling of the tongue layers. The LTR is symmetrical with an arrowhead shaped retractor head and anchor. Each end of the LTR mechanically hooks into soft tissue, preferably the connective tissue fascia of the mucosa and the midline septum. The hooking mechanism can be quite varied as many variations are known in the art. Non-limiting examples are: hooks, barbs, helixes, staples, screws, sutures, biointegrated permanent material, collagen, and elastin. However, the preferred example is as simple as possible: a short elastic shaft with a hook of firmer consistency at either end.

In alternative examples the implanted LTR can vary from 1 mm to 3 cm. Longer LTRs can couple the tongue base tissue to the boundary fascia between the tongue and genioglossus muscle, through the boundary layer to the genioglossus muscle, floor of mouth or mandible via barbs, hooks, fibrolic reaction, or other methods known in the art. The implant can be composed of biodegradable material that decomposes in a week to a year. Many materials used for surgical sutures can be adapted for this purpose.

Preferably the shaft is oriented such that the force on the retractor is at least one orientation that includes downward, forward, and to the side. Multiple implants may be used along the midline to distribute the coupling force without interfering with normal function. Depending on the anatomy of the patient implants may be inserted at any site in the tongue however, more preferable is the midline of the tongue and most preferable is the midline of the tongue curve. One or more of the following aspects of the invention can be used to mold the effects for the exact needs of the individual patient: implant site and orientation, shaft length and elasticity, and hook size, shape, and hardness.

Preferably the implant is bioresorbable over a period of 1 day to 10 years, more preferably 1 month to 1 year, most preferably 1 month to 6 months. The most preferable time range allows sufficient time for remodeling of the tongue and persistent if not permanent restoration of mechanical coupling in the area. Permanent implants are less preferable.

It is preferable that permanent or resorbable implants be inserted into superficial levels of the tongue in areas normally not undergoing great amount shape change during normal tongue activity. This minimizes the possibility of interference with normal function, particularly if there is an infection or fibrous reaction to the implant. To plan for atraumatic removal of the implant in cases of infection, pain or other complication, the implant should be designed to be easily removed without extensive surgery. To facilitate removal of the implant, the tear strength of the hooking mechanism should preferable range from 1 to 1000 gms. More preferably 10 to 100 grams. Preferably the arms of the hook would fold straight at these tear strength limits, allowing the implant to be removed without further damage to tissue as it is extracted.

### 6. Tongue Base Retraction (Figure 13)

Disclosed here is an example that focuses on retracting tissue of the tongue base, particularly the tongue base mucosa. This has the advantage that it is easy to insert by the physician, minimally invasive and easily adjustable by the patient.

In one example the device is inserted from one site to another on the superior surface of the tongue. The anterior part of the device is the anchor and the posterior part is the retractor. Tension between the two retracts the tongue surface and displaces the tongue base. Although the counter traction affects the anterior tongue surface it has no effect on normal function.

The shaft either passes directly underneath the mucosa (13. A) or takes a more direct line through the tongue (13. B). Passing the shaft directly underneath mucosa is easier for the physician. In this configuration the force at the retractor head is oriented laterally, and this causes the mucosa posterior to the retractor head to be pulled taut with some degree of indentation. In the more direct route the retraction force is oriented close to perpendicular to the tongue surface and there is more indentation then mucosal tension. The exact orientation at insertion can be varied to maximize the beneficial effects for the patient.

In another example the shaft reemerges in close proximity to the anchor and runs most of its course along the surface of the tongue. This has the advantage of avoiding even the minimally invasive tunnels formed by A and B. Furthermore the configurations can be combined and the shaft can travel the entire distance under the mucosa or can re-emerge one or more times (13. C).

In another example the anchor and/or the retractor can be embedded beneath mucosa and the shaft is detachable (13, D). In a preferred example the anchor/retractor would be a silastic disc 5 mm in diameter that is implanted under the mucosa. The disc has an 1 mm diameter extension that comes out of the pocket. The extension ends in a 2 mm disc (13. E, F). This extension reversibly couples to a shaft. Preferable shafts would be elastomeric. One preferable option would be a simple medical grade rubber band. Another option is a 1 x 1 mm strip of elastomeric material with expansion at either end to accommodate precut keyholes for attachment to the implanted anchor/retractor. These attachment holes would have 2 mm or greater inner holes to allow the stretched shaft to pass over the extension and 1 mm outer holes or clefts that slot into the extension (13. G). Materials could be pigmented to match the color of the tongue mucosa. A further example would allow the patient to depress the elevated extension so that it is flush with the mucosa, particularly when not in use (13. H). Many mechanisms are known in the art to allow reversible depression of a button like device.

A further example is an anchor member composed of an elastic sleeve slipped over the tongue blade (13. I). The shaft may be an integral part of the sleeve or a separate attachable component. The sleeve is preferably composed of silicone or other biocompatible elastomers. The distal end of the shaft can be reversibly attached to the implanted retractor head. The mechanism by which the shaft and implant are coupled may include elastic bands, clips, magnets of opposite polarity, and other mechanisms well known to those skilled in the art. The advantages of this arrangement are that only a small partially embedded implant is needed to achieve retraction.

In a further example two anchors are placed are in the PGFs (13. J). The anchors are attachment points for an elastic band passing over the base of tongue that serves to retract the base, or presses upon a smaller retractor component that is semi-implanted.

In a further embodiment an LTR anchored beneath the tongue blade with a shaft passes through the tongue blade to a intermediate anchor on the superior surface of the tongue. The shaft then passes posteriorly to a semi implanted retractor member (13. K). This allows adjustment of tension from the anchor site beneath the tongue blade.

In a further example a rigid shaft connects an anchor member below the tongue blade to a retractor member above the tongue blade. The retractor member is rotated forward by a sleeve that is reversibly placed over the tongue blade. The rotation of the retractor member, along with the rigid shaft, displaces the tissue of the tongue base along the midline (13. L)

### 7. Pharyngoglossal Fold

Disclosed here are methods and devices for using the PGF as a retractor or anchor site in order to beneficially effect the tongue, pharyngeal walls and/or soft palate.

On both sides of the tongue thin folds of mucosa connect the tongue to the mandible. These are called the pharyngoglossal folds (PGF). Within these folds are the palatoglossal, superior constrictor, styloglossus and hyoglossus muscles, from superior to inferior respectively. The PGFs separate the oral cavity (anterior) from the pharynx (posterior). Anterior to this attachment there is no lateral connection of the tongue and it is freely mobile. One of the muscles within the PGF is the palatoglossus which courses superiorly to connect with the soft palate, thereby forming what is seen in mouth as the anterior tonsillar pillar.

Unexpectedly the PGF has been found to have several advantages as a retraction site that enlarges the pharyngeal airspace. The connective tissue of the PGF is connected with that of the tongue. Therefore, it has been unexpectedly found that traction on the PGF is transmitted to the base of tongue. Moreover, as the superior pharyngeal constrictor and palatoglossus muscles are attached to the PGF and in turn connect with the lateral pharyngeal walls and soft palate these structures can also be retracted (Figure 14), In particular a preferred site within PGF is its superior end where many of these muscles overlap as they insert into the tongue. Therefore retraction at one site expands the pharyngeal airway by simultaneously stiffening and/or retracting the tongue base, lateral pharyngeal walls and soft palate. All of these effects have a beneficial effect on sleep disordered breathing.

A further advantage of the PGF is that it is easily accessible to both the physician and patient. The PGF is not normally seen during examination of the mouth as it in a folded state and hidden by the tongue surface above it. However, the PGF can be easily palpated by sliding a finger along the floor of the mouth next to the mandible, at the level of the edge of the mandible a smooth vertical wall is reached which blocks entry into the pharynx; this is the PGF. To visualize the PGF the tongue can be retracted medially with a tongue blade.

A further advantage of the PGF is that it does not have a lot of sensory innervation. The area of the mouth around the PGF is highly sensitive. Specifically, the tonsillar pillars and the tongue surface next to the PGF are the most sensitive areas of the upper airway that cause reflex gagging. However, it has unexpectedly been found that touching the PGF itself causes little or no gagging. Moreover, even the small amount of sensation caused by touching the PGF disappears within minutes.

A further advantage of the PGF is that it is thin and easy to puncture yet contains enough connective tissue to provide a firm interface with a retractor. Anatomical studies by the inventor have shown that the PGF has few neurovascular structures and is 1-3 mm in thickness. Therefore any piercing or puncturing of the PGF is safe.

It is possible to use an LTR to displace the PGF or neighboring tissue.

The most preferable retraction would be in an anterior direction as the retraction displaces the entire base of tongue anteriorly thereby increasing the retroglossal and retropalatal airspace. Also preferable is inferior retraction of the PGF as this displaces the tongue base inferiorly thereby removing tissue volume from the retropalatal area, the narrowest part of the upper airway. Lateral retraction would stiffen and flatten the posterior surface region of the tongue base. Less preferable is posterior or medial retraction.

This retraction could be unilateral but is preferably bilateral. This retraction could be acute, just during an obstructive episode, or semi acute, overnight while sleeping, or for extended durations. Extended durations of retraction would cause tissue remodeling that would cause the tongue to tend to remain in a more anterior position even without any force applied.

In one example an LTR is inserted across the PGF and passes across the frenulum to attach to a similarly implanted LTR on the opposite side (15B).

In one example the retractor lays against the PGF but the shaft passes through the tongue (15C) to an external anchor.

In one example the retractor is implanted within the PGF or neighboring tongue tissue and passes anteriorly and inferiorly to an implanted anchor in the tongue, genioglossus muscle, and/or floor of mouth (15D).

In one example the retractor is implanted against the superior PGF and the shaft passes inferiorly through or outside the PGF to an anchor that is implanted against the same PGF either on the same or opposite side (15E). This method retracts the superior PGF in an inferior direction.

In a further example against the PGF and the shaft passes medially and superiorly to an anchor on the superior surface of the tongue (15F).

In a further example the retractor is implanted at the tongue base and connects to two shafts that are placed at either PGF (16B).

In further example a shaft placed beneath the mucosa of the tongue base connects retractor members anterior to each PGF (16C).

In a further example, a shaft placed beneath the mucosa of the tongue base is connected at either end to implanted magnets in or around the PGFs (16D). External modified anchors with magnets of opposite polarity are used to bond to the implanted magnets and anchor them to external structures.

In a further preferred example, only the retractor member would be implanted in the PGF. The retractor would have a flange surface near its posterior aspect that would provide the interface against the PGF to cause anterior retraction. A second flange could be added anteriorly to prevent displacement of the conduit. A variety of coupling mechanisms could be used, Figure (16E) shows a magnetic mechanism.

Magnets are implanted within each PGF and external modified anchors with magnets of opposite polarity are used to bond to the implanted magnets and anchor them to external structures. Many other coupling mechanisms are known in the art, non-limiting examples being hooks, clamps or screws. This example is minimally invasive and allows the patient a very high degree of comfort during the day when the implant is unconnected and therefore unloaded. A variety of different shaft and anchor combinations can be tested without needing to replace the retractor implant.

Another option is a lateral dental anchor, this anchor may couple to the LTR using magnets, or mechanical mechanisms known in the art, The advantage of this device is that the PGF is very close to the mandibular teeth, and a secure but reversible loading of the implanted LTR can therefore be achieved with short devices. Moreover, the route from the PGF to the molar teeth is unlikely to cause the patient significant discomfort.

In still another option, anchors coupled to one of the following non-limiting list of structures insert into retractors on or lateral to the PGF or pass through the PGF to tongue structures: the styloglossus, hyoglossus, chondroglossus, pharyngeal constrictor, levator and tensor of the palate, masetter, temporalis, pterygoid, facial, and platysma muscles; the hyoid, mandible, facial, and vertebral bones; the thyroid, cricoid, epiglottic cartilages; the stylohyoid, ptyrogomandibular ligaments and other fascial structures.

It is to be understood that the exemplary embodiments are merely illustrative of the invention and that many variations of the above-described embodiments can be devised by one skilled in the art without departing from the scope of the invention. It is therefore intended that all such variations be included within the scope of the following claims.

## Claims

1. A tissue retractor for treatment of a breathing disorder, the tissue retractor comprising:
a) a shaft (S) sized for insertion into a soft tissue of the tongue located in a patient's oral cavity or pharynx;
b) a soft tissue retractor member (R, R/C) connected at a first end of the shaft; and
c) an anchor member (A) connected at a second end of the shaft, and **characterized by**
d) a bolster to be disposed between the external surface of the soft tissue of the tongue and the anchor member,
wherein at least one of the shaft, the retractor member, the bolster and the anchor member is to be positioned on an external surface of the soft tissue, and wherein the shaft is adjustable to vary a counterforce, the bolster configured to lengthen the shaft and increase the counterforce that prevents deformation of at least a portion of the soft tissue of the tongue to prevent obstruction of the patient's airway.

2. The tissue retractor of claim 1 wherein the bolster is substantially v-shaped with a concave surface adapted to complement the external surface of the soft tissue.

3. The tissue retractor of claim 2 wherein the bolster has a cleft, and at least a portion of the shaft is sized to fit within the cleft.

4. The tissue retractor of claim 1 wherein the anchor member is secured to a dental bolster.

5. The tissue retractor of claim 1 wherein the anchor member is reversibly attached to a modified anchor, and the modified anchor is fixed to a tissue located in the patient's oral cavity or pharynx.

6. The tissue retractor of claim 1 wherein the retractor member and the shaft are integrated as a single piece.

7. The tissue retractor of claim 1 wherein at least a portion of the shaft is adapted to be held inside a bore of a needle.

8. The tissue retraction of claim 1 wherein the retractor member has a plane at an angle substantially perpendicular to the shaft.

9. The tissue retractor of claim 6 wherein:
a) when undeployed the retractor member has a plane at an acute angle relative to the shaft; and
b) when deployed the retractor member is positioned on an external surface of the soft tissue with the plane at an angle substantially perpendicular to the shaft.

10. The tissue retractor of claim 1 wherein at least one of a first end of the shaft and a second end of the shaft is adapted to be reversibly coupled to the anchor.

11. The tissue retractor of claim 1 wherein the shaft comprises a distensible shaft and has a balloon portion, soft tissue movement being adapted to compress the balloon portion when the tissue retractor is fitted in use thereby lengthening the shaft.

12. The tissue retractor of claim 11 wherein an upper portion of the shaft adjacent to a first end of the shaft is distensible.

13. The tissue retractor of claim 1 further comprising the shaft with an upper portion that is distensible and wherein the upper portion of the shaft is adjacent the first end, and optionally or preferably wherein the upper portion comprises a balloon.

## Patentansprüche

1. Geweberetraktor zur Behandlung einer Atemstörung, wobei der Geweberetraktor aufweist:
a) einen Schaft (S), der größenmäßig zur Einführung in ein weiches Gewebe der Zunge in der Mundhöhle oder der Rachenhöhle eines Patienten ausgelegt ist;
b) ein Weichgewebe-Retraktorelement (R, R/C), das mit einem ersten Ende des Schaftes verbunden ist; und
c) ein Verankerungselement (A), das mit einem zweiten Ende des Schaftes verbunden ist, und **gekennzeichnet ist durch**
d) ein Polster, das zwischen der Außenfläche des weichen Gewebes der Zunge und dem Verankerungselement anzuordnen ist,
wobei der Schaft, das Retraktorelement, das Polster und/oder das Verankerungselement an der Außenfläche des weichen Gewebes zu positionieren sind, und wobei der Schaft justiert werden kann, um eine Gegenkraft zu variieren, wobei das Polster so gestaltet ist, dass es den Schaft verlängert und die Gegenkraft verstärkt, die eine Verformung von zumindest einem Abschnitt des weichen Gewebes der Zunge verhindert, um eine Obstruktion der Atemwegspassage des Patienten zu verhindern.

2. Geweberetraktor nach Anspruch 1, wobei das Polster im Wesentlichen v-förmig ist, mit einer konkaven Oberfläche, die so ausgelegt ist, dass sie der Form der Außenfläche des weichen Gewebes angepasst ist.

3. Geweberetraktor nach Anspruch 2, wobei das Polster einen Spalt aufweist und zumindest ein Abschnitt des Schaftes so bemessen ist, dass er in den Spalt passt.

4. Geweberetraktor nach Anspruch 1, wobei das Verankerungselement an einem Dentalpolster gesichert wird.

5. Geweberetraktor nach Anspruch 1, wobei das Verankerungselement lösbar an einem modifizierten Anker befestigt wird und der modifizierte Anker an einem Gewebe in der Mund- oder Rachenhöhle des Patienten fixiert wird.

6. Geweberetraktor nach Anspruch 1, wobei das Retraktorelement und der Schaft einstückig ausgebildet sind.

7. Geweberetraktor nach Anspruch 1, wobei zumindest ein Abschnitt des Schaftes dafür ausgelegt ist, innerhalb einer Bohrung einer Nadel gehalten zu werden.

8. Geweberetraktor nach Anspruch 1, wobei das Retraktorelement eine Ebene in einem Winkel aufweist, die im Wesentlichen senkrecht zum Schaft ist.

9. Geweberetraktor nach Anspruch 6, wobei:
a) das Retraktorelement, wenn es sich nicht aufgestellt hat, eine Ebene in einem spitzen Winkel in Bezug auf den Schaft aufweist; und
b) das Retraktorelement, wenn es sich aufgestellt hat, so an einer Außenfläche des weichen Gewebes positioniert ist, dass die Ebene einen Winkel aufweist, der im Wesentlichen senkrecht ist zum Schaft.

10. Geweberetraktor nach Anspruch 1, wobei ein erstes Ende des Schaftes und/oder ein zweites Ende des Schaftes für eine lösbare Befestigung am Anker ausgelegt sind.

11. Geweberetraktor nach Anspruch 1, wobei der Schaft einen dehnbaren Schaft umfasst und einen Ballonabschnitt aufweist, wobei eine Bewegung des weichen Gewebes geeignet ist, den Ballonabschnitt zusammenzudrücken, wenn der Geweberetraktor im Gebrauch angepasst wird, wodurch der Schaft verlängert wird.

12. Geweberetraktor nach Anspruch 11, wobei ein oberer Abschnitt des Schaftes angrenzend an ein erstes Ende des Schaftes dehnbar ist.

13. Geweberetraktor nach Anspruch 1, ferner den Schaft aufweisend mit einem oberen Abschnitt, der dehnbar ist, und wobei der obere Abschnitt des Schaftes an das erste Ende angrenzt, und wobei optional oder bevorzugt der obere Abschnitt einen Ballon umfasst.

## Revendications

1. Un écarteur de tissus pour le traitement d'un trouble respiratoire, l'écarteur de tissus comprenant:
a) une tige (S) dimensionnée pour une insertion dans un tissu mou de la langue situé dans une cavité orale ou le pharynx d'un patient,
b) un élément écarteur de tissus mous (R, R/C) raccordé au niveau d'une première extrémité de la tige, et
c) un élément d'ancrage (A) raccordé au niveau d'une deuxième extrémité de la tige, et **caractérisé par**
d) un appui à disposer entre la surface externe du tissu mou de la langue et l'élément d'ancrage,
où au moins un élément parmi la tige, l'élément écarteur, l'appui et l'élément d'ancrage doit être positionné sur une surface externe du tissu mou, et où la tige est ajustable de façon à varier une contre-force, l'appui étant configuré de façon à allonger la tige et accroître la contre-force qui empêche une déformation d'au moins une partie du tissu mou de la langue de façon à empêcher l'obstruction des voies aériennes du patient.

2. L'écarteur de tissus selon la Revendication 1 où l'appui est sensiblement en forme de V avec une surface concave adaptée de façon à compléter la surface externe du tissu mou.

3. L'écarteur de tissus selon la Revendication 2 où l'appui possède une échancrure, et au moins une partie de la tige est dimensionnée de façon à s'insérer à l'intérieur de l'échancrure.

4. L'écarteur de tissus selon la Revendication 1 où l'élément d'ancrage est fixé à un appui dentaire.

5. L'écarteur de tissus selon la Revendication 1 où l'élément d'ancrage est fixé de manière réversible à une ancre modifiée, et l'ancre modifiée est fixée à un tissu situé dans la cavité orale ou le pharynx d'un patient.

6. L'écarteur de tissus selon la Revendication 1 où l'élément écarteur et la tige sont intégrés sous la forme d'une pièce unique.

7. L'écarteur de tissus selon la Revendication 1 où au moins une partie de la tige est adaptée de façon à être maintenue à l'intérieur d'un chas d'une aiguille.

8. L'écarteur de tissus selon la Revendication 1 où l'élément écarteur possède un plan à un angle sensiblement perpendiculaire à la tige.

9. L'écarteur de tissus selon la Revendication 6 où :
a) lorsqu'il n'est pas déployé, l'élément écarteur possède un plan à un angle aigu par rapport à la tige, et
b) lorsqu'il est déployé, l'élément écarteur est positionné sur une surface externe du tissu mou avec le plan à un angle sensiblement perpendiculaire à la tige.

10. L'écarteur de tissus selon la Revendication 1 où au moins une extrémité parmi une première extrémité de la tige et une deuxième extrémité de la tige est adaptée de façon à être couplée de manière réversible à l'ancre.

11. L'écarteur de tissus selon la Revendication 1 où la tige comprend une tige extensible et possède une partie ballon, un déplacement du tissu mou étant adapté de façon à comprimer la partie ballon lorsque l'écarteur de tissus est installé en utilisation, allongeant ainsi la tige.

12. L'écarteur de tissus selon la Revendication 11 où une partie supérieure de la tige adjacente à une première extrémité de la tige est extensible.

13. L'écarteur de tissus selon la Revendication 1 comprenant en outre la tige avec une partie supérieure qui est extensible et où la partie supérieure de la tige est adjacente à la première extrémité, et éventuellement ou de préférence où la partie supérieure comprend un ballon.
